# EUROPÄISCHE PATENTANMELDUNG

(11) **EP 4 685 233 A1**
(43) Veröffentlichungstag der Anmeldung: **28.01.2026**
(21) Anmeldenummer: 25181879.5
(22) Anmeldetag: 10.06.2025
(51) Int. Cl.: C12N 9/54, C11D 3/386, C12N 9/64

(54) **PROTEASE-VARIANTEN MIT VERBESSERTER LAGERSTABILITÄT UND VERWENDUNG IN REINIGUNGSMITTEL**

(30) Priorität: 26.07.2024 DE 102024207057
(71) Anmelder: Henkel AG & Co. KGaA, 40589 Düsseldorf (DE)
(72) Erfinder: Falkenberg, Fabian, 41460 Neuss (DE); Degering, Christian, 40699 Erkrath (DE); Mackenberg, Pascal, 40225 Düsseldorf (DE); van Lier, Margret, 40721 Hilden (DE)

(57) **Zusammenfassung**

Die Erfindung betrifft Proteasen, die proteolytische Aktivität aufweisen und eine Aminosäuresequenz umfassen, die zu der in SEQ ID NO:1 angegebenen Aminosäuresequenz über deren Gesamtlänge zu mindestens 70% identisch sind, wobei die Proteasen, jeweils bezogen auf die Nummerierung gemäß SEQ ID NO:1, an mindestens einer der Positionen, die die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweisen. Solche Proteasen sind für den Einsatz in Wasch- und Reinigungsmitteln, insbesondere Textilwaschmitteln, geeignet und weisen eine gegenüber einer Referenzprotease verbesserte Lagerstabilität auf. Die Erfindung betrifft ferner die Verwendung dieser Proteasen und Verfahren, in denen sie eingesetzt werden, sowie diese enthaltende Wasch- und Reinigungsmittel, insbesondere Textilwaschmittel.

## Beschreibung

Die Erfindung liegt auf dem Gebiet der Enzymtechnologie. Die Erfindung betrifft Proteasen, deren Aminosäuresequenz insbesondere im Hinblick auf den Einsatz in Wasch- und Reinigungsmitteln, insbesondere im Hinblick auf Textilwaschmittel, verändert wurden, um ihre Lagerstabilität zu verbessern, und die für sie codierende Nukleinsäuren sowie deren Herstellung. Die Erfindung betrifft ferner die Verwendung dieser Proteasen und Verfahren, in denen sie eingesetzt werden, sowie diese enthaltende Wasch- und Reinigungsmittel, insbesondere Textilwaschmittel.

Proteasen gehören zu den technisch bedeutendsten Enzymen überhaupt. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet z.B. der Artikel "Subtilases: Subtilisin-like Proteases" von R. Siezen, Seiten 75-95 in "Subtilisin enzymes", herausgegeben von R. Bott und C. Betzel, New York, 1996. Subtilasen werden natürlicherweise von Mikroorganismen gebildet. Hierunter sind insbesondere die von *Bacillus-*Spezies gebildeten und sezernierten Subtilisine als bedeutendste Gruppe innerhalb der Subtilasen zu erwähnen.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die alkalische Protease aus *Bacillus lentus,* insbesondere aus *Bacillus lentus* DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so z.B. für den Einsatz in Wasch- und Reinigungsmitteln optimiert. Dazu gehören Punkt-, Deletions- oder Insertionsmutagenese oder Fusion mit anderen Proteinen oder Proteinteilen. So sind für die meisten aus dem Stand der Technik bekannten Proteasen entsprechend optimierte Varianten bekannt.

Generell sind nur ausgewählte Proteasen für den Einsatz in flüssigen tensidhaltigen Zubereitungen überhaupt geeignet. Viele Proteasen zeigen in derartigen Zubereitungen keine ausreichende katalytische Leistung oder aber sie sind nicht ausreichend stabil. Für die Anwendung von Proteasen in Wasch- und Reinigungsmitteln ist daher eine hohe katalytische Aktivität und Stabilität unter Bedingungen, wie sie sich während eines Waschvorgangs darstellen, besonders wünschenswert. Folglich haben Protease- und Tensid-haltige flüssige Formulierungen aus dem Stand der Technik den Nachteil, dass die enthaltenen Proteasen unter Standardreinigungsbedingungen keine zufriedenstellende proteolytische Aktivität aufweisen und/oder nicht ausreichend lagerstabil sind, und die Formulierungen daher keine optimale Reinigungsleistung an proteasesensitiven Anschmutzungen zeigen. Proteasesensitive Anschmutzungen sind vorzugsweise aus der aus blut-, ei(gelb)-, milch- und anderen proteinhaltigen Anschmutzungen bestehenden Gruppe ausgewählt.

Es besteht weiterhin Bedarf, die Lagerstabilität von enzymhaltigen, insbesondere proteasehaltigen, Wasch- und Reinigungsmitteln, insbesondere Textilwaschmitteln, zu verbessern, insbesondere hinsichtlich der Reinigungsleistung an proteasesensitiven Anschmutzungen, insbesondere in einem Temperaturbereich von etwa 20°C bis etwa 40°C. Ferner besteht weiterhin Bedarf, die Lagerstabilität der Protease in einem proteasehaltigen Wasch- und Reinigungsmittel, insbesondere Textilwaschmittel, zu verbessern.

Überraschenderweise wurde jetzt festgestellt, dass eine Protease aus *Halalkalibacter okhensis Kh10-101oder* eine hierzu hinreichend ähnliche Protease (bezogen auf die Sequenzidentität), die bezogen auf die Nummerierung gemäß SEQ ID NO:1 an mindestens einer der Positionen, die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist, hinsichtlich ihrer Lagerstabilität gegenüber einer Referenzprotease (Wildtyp gemäß SEQ ID NO:1) verbessert ist, und daher besonders für den Einsatz in Wasch- und Reinigungsmitteln, insbesondere Textilwaschmitteln, geeignet ist.

Gegenstand der Erfindung ist daher eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens einer der Positionen, die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist.

Ein bevorzugter Gegenstand der Erfindung ist eine Protease, die proteolytische Aktivität aufweist und eine Aminosäuresubstitutionskombination aufweist, die aus der aus (i) A209V-N237P, (ii) A209V, (iii) N121F-A209V-N237P, (iv) A194C-N2181-N237W, (v) A209V-N237W und (vi) A209V-N218I-N237P bestehenden Gruppe ausgewählt ist, jeweils bezogen auf die Nummerierung gemäß SEQ ID NO:1.

Ein weiterer Gegenstand der Erfindung ist ein Verfahren zur Herstellung einer Protease, umfassend das Einbringen mindestens einer Aminosäuresubstitution an mindestens einer der Positionen, die bezogen auf die Nummerierung gemäß SEQ ID NO:1 den Positionen 121, 194, 209, 218 und 237 entsprechen, ausgewählt aus der aus N121F, A194C, A209V, T218I, N237P und N237W bestehenden Gruppe, in ein Ausgangsmolekül, das eine Aminosäuresequenz aufweist, die mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% oder 100% Sequenzidentität mit der in SEQ ID NO:1 angegebenen Aminosäuresequenz über deren Gesamtlänge aufweist.

Eine Protease im Sinne der vorliegenden Patentanmeldung umfasst daher sowohl die Protease als solche als auch eine mit einem erfindungsgemäßen Verfahren hergestellte Protease. Alle Ausführungen zur Protease beziehen sich daher sowohl auf die Protease als solche wie auch auf die mittels entsprechender Verfahren hergestellten Proteasen, sowie auf die entsprechenden Verfahren, insbesondere Herstellungsverfahren der Protease.

Weitere Aspekte der Erfindung betreffen die für diese Proteasen codierenden Nukleinsäuren, erfindungsgemäße Proteasen oder Nukleinsäuren enthaltende nicht-menschliche Wirtszellen sowie erfindungsgemäße Proteasen umfassende Wasch- und Reinigungsmittel, insbesondere Textilwaschmittel, Wasch- und Reinigungsverfahren, und Verwendung einer erfindungsgemäßen Protease in einem Wasch- oder Reinigungsmittel, insbesondere Textilwaschmittel, zur Entfernung mindestens einer proteasesensitiven Anschmutzung.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Alle Prozentangaben sind, sofern nicht anders angegeben, Gewichtsprozent (Gew.-%).

Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

"Mindestens ein(e)", wie hierin verwendet, bedeutet ein(e) oder mehrere, d.h. 1, 2, 3, 4, 5, 6, 7, 8, 9, 10, 11, 12, 13, 14 oder mehr.

Der Begriff "Wasch- und Reinigungsmittel" bzw. "Wasch- oder Reinigungsmittel", wie hierin verwendet, ist gleichbedeutend mit dem Begriff "Mittel" und bezeichnet eine Zusammensetzung zum Reinigen von Textilien und/oder harten Oberflächen, insbesondere Geschirr, wie in der Beschreibung erläutert.

"Etwa", "ca." oder "ungefähr", wie hierin in Bezug auf einen Zahlenwert verwendet, beziehen sich auf den entsprechenden Zahlenwert ±10%, vorzugsweise ±5%.

"Im Wesentlichen frei von" bedeutet, dass die Zusammensetzung bzw. das Mittel weniger als 2 Gew.-%, vorzugsweise weniger als 1 Gew.-%, weiter bevorzugt weniger als 0,5 Gew.-% und besonders bevorzugt weniger als 0,1 Gew.-%, der entsprechenden Substanz, bezogen auf das Gesamtgewicht der Zusammensetzung/des Mittels, enthält.

"Flüssig", wie hierin verwendet, schließt Flüssigkeiten und Gele sowie auch pastöse Zusammensetzungen ein. Es ist bevorzugt, dass die flüssigen Zusammensetzungen bei Raumtemperatur fließfähig und gießbar sind, es ist aber auch möglich, dass sie eine Fließgrenze aufweisen.

Ein Stoff, z.B. eine Zusammensetzung oder ein Mittel ist gemäß Definition der Erfindung festförmig, wenn sie bei 25°C und 1013 mbar im festen Aggregatzustand vorliegt.

Ein Stoff, z.B. eine Zusammensetzung oder ein Mittel ist gemäß Definition der Erfindung flüssig, wenn sie bei 25°C und 1013 mbar im flüssigen Aggregatzustand vorliegt. Dabei umfasst flüssig auch gelförmig.

"Variante", wie hierin verwendet, bezieht sich auf natürliche oder artifiziell erzeugte Variationen einer nativen Protease, die eine gegenüber der Referenzform abgewandelte Aminosäuresequenz aufweist.

Eine "Verbesserung der Stabilität eines Enzyms" im Sinne der Erfindung liegt vor, wenn die Anwesenheit einer hierin definierten Aminosäuresubstitution bewirkt, dass eine Protease mit einer solchen Aminosäureveränderung nach Lagerung in Wasch- und/oder Reinigungsmittel, insbesondere Textilwaschmittel, eine höhere enzymatische Aktivität der Protease und/oder ggf. weiterer in dem Wasch- und/oder Reinigungsmittel enthaltenen Enzyme aufweist im Vergleich zu einer Kontrollzubereitung, die eine Protease ohne eine erfindungsgemäße Aminosäureveränderung umfasst. Nach Lagerung in Wasch- und/oder Reinigungsmittel, insbesondere Textilwaschmittel, weist eine erfindungsgemäße Protease eine höhere Restaktivität auf als eine Referenzprotease (z.B. die Wildtyp-Protease), wobei die Proteasen auf die gleiche Art und Weise behandelt werden, insbesondere betreffend die Bedingungen der Lagerung und die Bestimmung der Enzymaktivität. Zunehmend bevorzugt erfolgt die Lagerung für mindestens 1 Woche, 2 Wochen, 3 Wochen oder 4 Wochen. Weiter bevorzugt erfolgt die Lagerung bei einer Temperatur von 20°C, 25°C, 30°C oder 40°C.

Der Begriff "Textil", wie hierin verwendet, bezeichnet jedes textile Material, einschließlich Garne, Garnvorprodukte, Fasern, Vliesstoffe, natürliche Materialien, synthetische Materialien und alle anderen textilen Materialien, Gewebe aus diesen Materialien und aus Geweben hergestellte Produkte (z.B. Kleidungsstücke und andere Artikel). Das Textil oder Gewebe kann in Form von Gewirken, Geweben, Denims, Vliesstoffen, Filzen, Garnen und Frottee vorliegen. Das Textil kann auf Cellulose basieren, wie z.B. natürliche Cellulosefasern wie Baumwolle, Flachs/Leinen, Jute, Ramie, Sisal oder Kokosfasern, oder künstlich hergestellte Zellulosefasern (z.B. aus Zellstoff) wie Viskose/Rayon, Celluloseacetatfasern (Tricell), Lyocell oder Mischungen davon. Das Textil oder Gewebe kann auch aus Nicht-Cellulosefasern bestehen, z.B. aus natürlichen Polyamiden wie Wolle, Kamel, Kaschmir, Mohair, Kaninchen und Seide oder aus synthetischen Polymeren wie Nylon, Aramid, Polyester, Acryl, Polypropylen und Spandex/Elasthan oder Mischungen daraus sowie aus Mischungen von Cellulosefasern und Nicht-Cellulosefasern. Beispiele für Mischungen sind Mischungen aus Baumwolle und/oder Rayon/Viskose mit einem oder mehreren Begleitmaterialien wie Wolle, synthetischen Fasern (z.B. Polyamidfasern, Acrylfasern, Polyesterfasern, Polyvinylchloridfasern, Polyurethanfasern, Polyharnstofffasern, Aramidfasern) und/oder cellulosehaltigen Fasern (z.B. Rayon/Viskose, Ramie, Flachs/Leinen, Jute, Celluloseacetatfasern, Lyocell). Bei dem Gewebe kann es sich um herkömmliche waschbare Wäsche handeln, z.B. um verschmutzte Haushaltswäsche. Wenn der Begriff "Gewebe" oder "Kleidungsstück" verwendet wird, soll er auch den weiter gefassten Begriff "Textilien" umfassen.

Die vorliegende Erfindung basiert auf der überraschenden Erkenntnis der Erfinder, dass Aminosäuresubstitutionen an den hierin beschriebenen Positionen eine verbesserte Lagerstabilität dieser veränderten Protease in Wasch- und Reinigungsmitteln, insbesondere Textilwaschmitteln, bewirkt.

In bevorzugten Ausführungsformen führen die erfindungsgemäßen Veränderungen an mindestens einer der Positionen, die den Positionen 121, 194, 209, 218 und 237 entsprechen, insbesondere mindestens eine Aminosäuresubstitution, die aus der aus N121F, A194C, A209V, T218I, N237P und N237W bestehenden Gruppe ausgewählt ist, zu einer verbesserten Lagerstabilität dieser veränderten Protease in Wasch- und Reinigungsmitteln, insbesondere Textilwaschmitteln.

In bevorzugten Ausführungsformen führen die erfindungsgemäßen Veränderungen an mindestens einer der Positionen, die den Positionen 121, 194, 209, 218 und 237 entsprechen, insbesondere mindestens eine Aminosäuresubstitution, die aus der aus N121F, A194C, A209V, T218I, N237P und N237W bestehenden Gruppe ausgewählt ist, zu einer verbesserten Lagerstabilität dieser veränderten Protease in Wasch- und Reinigungsmitteln, insbesondere Textilwaschmitteln, wenn das Wasch- und Reinigungsmittel, insbesondere Textilwaschmittel, für mindestens 1 Woche, 2 Wochen, 3 Wochen oder 4 Wochen bei einer Temperatur von 20°C, 25°C, 30°C oder 40°C gelagert wird.

In bevorzugten Ausführungsformen führen die erfindungsgemäßen Veränderungen an mindestens einer der Positionen, die den Positionen 121, 194, 209, 218 und 237 entsprechen, insbesondere mindestens eine Aminosäuresubstitution, die aus der aus N121F, A194C, A209V, T218I, N237P und N237W bestehenden Gruppe ausgewählt ist, zu einer verbesserten Lagerstabilität dieser veränderten Protease in Wasch- und Reinigungsmitteln, insbesondere Textilwaschmitteln, wenn die Lagerstabilität wie in Beispiel 1 beschrieben bestimmt wird.

Das ist insbesondere insoweit überraschend, als dass bisher keine Protease mit solchen Veränderungen für die Verwendung in Wasch- und Reinigungsmitteln, insbesondere Textilwaschmitteln, beschrieben wurden. Insbesondere wurden solche erfindungsgemäß veränderten Proteasen nicht im Zusammenhang mit einer verbesserten Lagerstabilität beschrieben.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens einer der Positionen, die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens zwei der Positionen, die den Positionen 121, 194, 209, 218 und 237 entsprechen, mindestens zwei Aminosäuresubstitutionen, die aus der aus N121F, A194C, A209V, T218I, N237P und N237W bestehenden Gruppe ausgewählt sind, aufweist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens drei der Positionen, die den Positionen 121, 194, 209, 218 und 237 entsprechen, mindestens drei Aminosäuresubstitutionen, die aus der aus N121F, A194C, A209V, T218I, N237P und N237W bestehenden Gruppe ausgewählt sind, aufweist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens vier der Positionen, die den Positionen 121, 194, 209, 218 und 237 entsprechen, mindestens vier Aminosäuresubstitutionen, die aus der aus N121F, A194C, A209V, T218I, N237P und N237W bestehenden Gruppe ausgewählt sind, aufweist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens fünf der Positionen, die den Positionen 121, 194, 209, 218 und 237 entsprechen, mindestens fünf Aminosäuresubstitutionen, die aus der aus N121F, A194C, A209V, T218I, N237P und N237W bestehenden Gruppe ausgewählt sind, aufweist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, und jeweils bezogen auf die Nummerierung gemäß SEQ ID NO:1, eine Aminosäuresubstitutionskombination aufweist, die aus der aus (i) A209V-N237P, (ii) A209V, (iii) N121F-A209V-N237P, (iv) A194C-N218I-N237W, (v) A209V-N237W und (vi) A209V-N218I-N237P bestehenden Gruppe ausgewählt ist.

In besonders bevorzugten Ausführungsformen umfasst die erfindungsgemäße Protease eine Aminosäuresubstitutionskombination, die aus der aus (i) A209V-N237P, (ii) A209V, (iii) N121F-A209V-N237P, (iv) A194C-N218I-N237W, (v) A209V-N237W und (vi) A209V-N218I-N237P bestehenden Gruppe ausgewählt ist, wobei die Nummerierung jeweils bezogen auf die Nummerierung gemäß SEQ ID NO:1 ist und wobei die Protease neben den genannten Aminosäuresubstitutionen keine weiteren Änderungen umfasst.

In bevorzugten Ausführungsformen führen die hierin beschriebenen erfindungsgemäßen Aminosäuresubstitutionen zu einer verbesserten Lagerstabilität dieser veränderten Protease in Wasch- und Reinigungsmitteln, insbesondere Textilwaschmitteln. Erfindungsgemäße Proteasen weisen folglich nach Lagerung in Wasch- und Reinigungsmitteln, insbesondere Textilwaschmitteln, eine höhere Restaktivität auf als eine Referenzprotease, insbesondere Wildtyp-Protease gemäß SEQ ID NO:1, und ermöglichen folglich eine verbesserte Entfernung von mindestens einer, bevorzugt mehreren, proteasesensitiven Anschmutzungen auf Textilien und/oder harten Oberflächen, insbesondere Geschirr, nach Lagerung in Wasch- und Reinigungsmitteln, insbesondere Textilwaschmitteln. Typische proteasesensitive Anschmutzungen umfassen z.B. ei(gelb)-, blut-, milch- und andere proteinhaltige Anschmutzungen. Besonders bevorzugt ermöglichen erfindungsgemäße Proteasen eine verbesserte Entfernung von ei(gelb)-haltigen Anschmutzungen auf Textilien und/oder harten Oberflächen, insbesondere Geschirr. Eine erfindungsgemäße Verbesserung der Reinigungsleistung, insbesondere der proteolytischen Reinigungsleistung, liegt dann vor, wenn die Protease an mindestens einer proteasesensitiven Anschmutzung, die vorzugsweise aus der aus blut-, ei(gelb)-, milch- und anderen proteinhaltigen Anschmutzungen bestehenden Gruppe ausgewählt ist, eine verbesserte Reinigungsleistung gegenüber einer Referenzprotease, insbesondere Wildtyp-Protease gemäß SEQ ID NO:1, zeigt, bevorzugt die Reinigungsleistung nach Lagerung. Eine Erhöhung der Stabilität während der Lagerung und/oder während des Einsatzes, z.B. während des Waschprozesses, führt dazu, dass die enzymatische Aktivität länger anhält und damit die Reinigungsleistung verbessert wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens einer der Positionen, die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist, wobei die Protease eine verbesserte Lagerstabilität gegenüber einer Referenzprotease (insbesondere Wildtyp-Protease gemäß SEQ ID NO:1) zeigt.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens zwei der Positionen, die den Positionen 121, 194, 209, 218 und 237 entsprechen, mindestens zwei Aminosäuresubstitutionen, die aus der aus N121F, A194C, A209V, T218I, N237P und N237W bestehenden Gruppe ausgewählt sind, aufweist, wobei die Protease eine verbesserte Lagerstabilität gegenüber einer Referenzprotease (insbesondere Wildtyp-Protease gemäß SEQ ID NO:1) zeigt.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens drei der Positionen, die den Positionen 121, 194, 209, 218 und 237 entsprechen, mindestens drei Aminosäuresubstitutionen, die aus der aus N121F, A194C, A209V, T218I, N237P und N237W bestehenden Gruppe ausgewählt sind, aufweist, wobei die Protease eine verbesserte Lagerstabilität gegenüber einer Referenzprotease (insbesondere Wildtyp-Protease gemäß SEQ ID NO:1) zeigt.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens vier der Positionen, die den Positionen 121, 194, 209, 218 und 237 entsprechen, mindestens vier Aminosäuresubstitutionen, die aus der aus N121F, A194C, A209V, T218I, N237P und N237W bestehenden Gruppe ausgewählt sind, aufweist, wobei die Protease eine verbesserte Lagerstabilität gegenüber einer Referenzprotease (insbesondere Wildtyp-Protease gemäß SEQ ID NO:1) zeigt.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens fünf der Positionen, die den Positionen 121, 194, 209, 218 und 237 entsprechen, mindestens fünf Aminosäuresubstitutionen, die aus der aus N121F, A194C, A209V, T218I, N237P und N237W bestehenden Gruppe ausgewählt sind, aufweist, wobei die Protease eine verbesserte Lagerstabilität gegenüber einer Referenzprotease (insbesondere Wildtyp-Protease gemäß SEQ ID NO:1) zeigt.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, eine Aminosäuresubstitutionskombination aufweist, die aus der aus (i) A209V-N237P, (ii) A209V, (iii) N121F-A209V-N237P, (iv) A194C-N218I-N237W, (v) A209V-N237W und (vi) A209V-N218I-N237P bestehenden Gruppe ausgewählt ist, wobei die Protease eine verbesserte Lagerstabilität gegenüber einer Referenzprotease (insbesondere Wildtyp-Protease gemäß SEQ ID NO:1) zeigt.

In bevorzugten Ausführungsformen ist die erfindungsgemäße Protease eine Protease, die proteolytische Aktivität aufweist und eine Aminosäuresubstitutionskombination aufweist, die aus der aus (i) A209V-N237P, (ii) A209V, (iii) N121F-A209V-N237P, (iv) A194C-N218I-N237W, (v) A209V-N237W und (vi) A209V-N218I-N237P bestehenden Gruppe ausgewählt ist, wobei die Nummerierung jeweils bezogen auf die Nummerierung gemäß SEQ ID NO:1 ist, wobei die Protease neben den genannten Aminosäuresubstitutionen keine weiteren Änderungen umfasst, und wobei die Protease eine verbesserte Lagerstabilität gegenüber einer Referenzprotease (insbesondere Wildtyp-Protease gemäß SEQ ID NO:1) zeigt.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens einer der Positionen, die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist, wobei die Protease eine verbesserte Lagerstabilität gegenüber einer Referenzprotease (insbesondere Wildtyp-Protease gemäß SEQ ID NO:1) zeigt, wenn das Wasch- und Reinigungsmittel, insbesondere Textilwaschmittel, für mindestens 1 Woche, 2 Wochen, 3 Wochen oder 4 Wochen bei einer Temperatur von 20°C, 25°C, 30°C oder 40°C gelagert wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens zwei der Positionen, die den Positionen 121, 194, 209, 218 und 237 entsprechen, mindestens zwei Aminosäuresubstitutionen, die aus der aus N121F, A194C, A209V, T218I, N237P und N237W bestehenden Gruppe ausgewählt sind, aufweist, wobei die Protease eine verbesserte Lagerstabilität gegenüber einer Referenzprotease (insbesondere Wildtyp-Protease gemäß SEQ ID NO:1) zeigt, wenn das Wasch- und Reinigungsmittel, insbesondere Textilwaschmittel, für mindestens 1 Woche, 2 Wochen, 3 Wochen oder 4 Wochen bei einer Temperatur von 20°C, 25°C, 30°C oder 40°C gelagert wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens drei der Positionen, die den Positionen 121, 194, 209, 218 und 237 entsprechen, mindestens drei Aminosäuresubstitutionen, die aus der aus N121F, A194C, A209V, T218I, N237P und N237W bestehenden Gruppe ausgewählt sind, aufweist, wobei die Protease eine verbesserte Lagerstabilität gegenüber einer Referenzprotease (insbesondere Wildtyp-Protease gemäß SEQ ID NO:1) zeigt, wenn das Wasch- und Reinigungsmittel, insbesondere Textilwaschmittel, für mindestens 1 Woche, 2 Wochen, 3 Wochen oder 4 Wochen bei einer Temperatur von 20°C, 25°C, 30°C oder 40°C gelagert wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens vier der Positionen, die den Positionen 121, 194, 209, 218 und 237 entsprechen, mindestens vier Aminosäuresubstitutionen, die aus der aus N121F, A194C, A209V, T218I, N237P und N237W bestehenden Gruppe ausgewählt sind, aufweist, wobei die Protease eine verbesserte Lagerstabilität gegenüber einer Referenzprotease (insbesondere Wildtyp-Protease gemäß SEQ ID NO:1) zeigt, wenn das Wasch- und Reinigungsmittel, insbesondere Textilwaschmittel, für mindestens 1 Woche, 2 Wochen, 3 Wochen oder 4 Wochen bei einer Temperatur von 20°C, 25°C, 30°C oder 40°C gelagert wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens fünf der Positionen, die den Positionen 121, 194, 209, 218 und 237 entsprechen, mindestens fünf Aminosäuresubstitutionen, die aus der aus N121F, A194C, A209V, T218I, N237P und N237W bestehenden Gruppe ausgewählt sind, aufweist, wobei die Protease eine verbesserte Lagerstabilität gegenüber einer Referenzprotease (insbesondere Wildtyp-Protease gemäß SEQ ID NO:1) zeigt, wenn das Wasch- und Reinigungsmittel, insbesondere Textilwaschmittel, für mindestens 1 Woche, 2 Wochen, 3 Wochen oder 4 Wochen bei einer Temperatur von 20°C, 25°C, 30°C oder 40°C gelagert wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, eine Aminosäuresubstitutionskombination aufweist, die aus der aus (i) A209V-N237P, (ii) A209V, (iii) N121F-A209V-N237P, (iv) A194C-N218I-N237W, (v) A209V-N237W und (vi) A209V-N218I-N237P bestehenden Gruppe ausgewählt ist, wobei die Protease eine verbesserte Lagerstabilität gegenüber einer Referenzprotease (insbesondere Wildtyp-Protease gemäß SEQ ID NO:1) zeigt, wenn das Wasch- und Reinigungsmittel, insbesondere Textilwaschmittel, für mindestens 1 Woche, 2 Wochen, 3 Wochen oder 4 Wochen bei einer Temperatur von 20°C, 25°C, 30°C oder 40°C gelagert wird.

In bevorzugten Ausführungsformen ist die erfindungsgemäße Protease eine Protease, die proteolytische Aktivität aufweist und eine Aminosäuresubstitutionskombination aufweist, die aus der aus (i) A209V-N237P, (ii) A209V, (iii) N121F-A209V-N237P, (iv) A194C-N218I-N237W, (v) A209V-N237W und (vi) A209V-N218I-N237P bestehenden Gruppe ausgewählt ist, wobei die Nummerierung jeweils bezogen auf die Nummerierung gemäß SEQ ID NO:1 ist, wobei die Protease neben den genannten Aminosäuresubstitutionen keine weiteren Änderungen umfasst, und wobei die Protease eine verbesserte Lagerstabilität gegenüber einer Referenzprotease (insbesondere Wildtyp-Protease gemäß SEQ ID NO:1) zeigt, wenn das Wasch- und Reinigungsmittel, insbesondere Textilwaschmittel, für mindestens 1 Woche, 2 Wochen, 3 Wochen oder 4 Wochen bei einer Temperatur von 20°C, 25°C, 30°C oder 40°C gelagert wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens einer der Positionen, die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist, wobei die Protease eine verbesserte Lagerstabilität gegenüber einer Referenzprotease (insbesondere Wildtyp-Protease gemäß SEQ ID NO:1) zeigt, wenn die Lagerstabilität wie in Beispiel 1 beschrieben bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens zwei der Positionen, die den Positionen 121, 194, 209, 218 und 237 entsprechen, mindestens zwei Aminosäuresubstitutionen, die aus der aus N121F, A194C, A209V, T218I, N237P und N237W bestehenden Gruppe ausgewählt sind, aufweist, wobei die Protease eine verbesserte Lagerstabilität gegenüber einer Referenzprotease (insbesondere Wildtyp-Protease gemäß SEQ ID NO:1) zeigt, wenn die Lagerstabilität wie in Beispiel 1 beschrieben bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens drei der Positionen, die den Positionen 121, 194, 209, 218 und 237 entsprechen, mindestens drei Aminosäuresubstitutionen, die aus der aus N121F, A194C, A209V, T218I, N237P und N237W bestehenden Gruppe ausgewählt sind, aufweist, wobei die Protease eine verbesserte Lagerstabilität gegenüber einer Referenzprotease (insbesondere Wildtyp-Protease gemäß SEQ ID NO:1) zeigt, wenn die Lagerstabilität wie in Beispiel 1 beschrieben bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens vier der Positionen, die den Positionen 121, 194, 209, 218 und 237 entsprechen, mindestens vier Aminosäuresubstitutionen, die aus der aus N121F, A194C, A209V, T218I, N237P und N237W bestehenden Gruppe ausgewählt sind, aufweist, wobei die Protease eine verbesserte Lagerstabilität gegenüber einer Referenzprotease (insbesondere Wildtyp-Protease gemäß SEQ ID NO:1) zeigt, wenn die Lagerstabilität wie in Beispiel 1 beschrieben bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens fünf der Positionen, die den Positionen 121, 194, 209, 218 und 237 entsprechen, mindestens fünf Aminosäuresubstitutionen, die aus der aus N121F, A194C, A209V, T218I, N237P und N237W bestehenden Gruppe ausgewählt sind, aufweist, wobei die Protease eine verbesserte Lagerstabilität gegenüber einer Referenzprotease (insbesondere Wildtyp-Protease gemäß SEQ ID NO:1) zeigt, wenn die Lagerstabilität wie in Beispiel 1 beschrieben bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, eine Aminosäuresubstitutionskombination aufweist, die aus der aus (i) A209V-N237P, (ii) A209V, (iii) N121F-A209V-N237P, (iv) A194C-N218I-N237W, (v) A209V-N237W und (vi) A209V-N218I-N237P bestehenden Gruppe ausgewählt ist, wobei die Protease eine verbesserte Lagerstabilität gegenüber einer Referenzprotease (insbesondere Wildtyp-Protease gemäß SEQ ID NO:1) zeigt, wenn die Lagerstabilität wie in Beispiel 1 beschrieben bestimmt wird.

In bevorzugten Ausführungsformen ist die erfindungsgemäße Protease eine Protease, die proteolytische Aktivität aufweist und eine Aminosäuresubstitutionskombination aufweist, die aus der aus (i) A209V-N237P, (ii) A209V, (iii) N121F-A209V-N237P, (iv) A194C-N218I-N237W, (v) A209V-N237W und (vi) A209V-N218I-N237P bestehenden Gruppe ausgewählt ist, wobei die Nummerierung jeweils bezogen auf die Nummerierung gemäß SEQ ID NO:1 ist, wobei die Protease neben den genannten Aminosäuresubstitutionen keine weiteren Änderungen umfasst, und wobei die Protease eine verbesserte Lagerstabilität gegenüber einer Referenzprotease (insbesondere Wildtyp-Protease gemäß SEQ ID NO:1) zeigt, wenn die Lagerstabilität wie in Beispiel 1 beschrieben bestimmt wird.

Erfindungsgemäße Proteasen verfügen über eine katalytische Aktivität in Wasch- und/oder Reinigungsmitteln. In vielfältigen Ausführungsformen können die erfindungsgemäßen Proteasen eine proteolytische Aktivität besitzen, die bezogen auf den Wildtyp (SEQ ID NO:1) mindestens 101%, 102%, 103%, 104%, 105%, 106%, 107%, 108%, 109%, 110%, 111%, 112%, 113%, 114%, 115%, 116%, 117%, 118%, 119%, 120%, 121%, 122%, 123%, 124%, 125%, 126%, 127%, 128%, 129%, 130% oder mehr beträgt.

Die erfindungsgemäßen Proteasen verfügen über eine gegenüber einer Referenzprotease (insbesondere Wildtyp-Protease gemäß SEQ ID NO:1) erhöhte Stabilität in Wasch- und Reinigungsmitteln, insbesondere Textilwaschmitteln, z.B. gegenüber Tensiden und/oder Bleichmitteln und/oder Chelatoren, und/oder gegenüber Temperatureinflüssen, insbesondere gegenüber hohen Temperaturen, und/oder gegenüber sauren oder alkalischen Bedingungen und/oder gegenüber pH-Wert-Änderungen und/oder gegenüber denaturierenden oder oxidierenden Agentien und/oder gegenüber (auto)proteolytischem Abbau und/oder gegenüber einer Veränderung der Redox-Verhältnisse. Mit besonders bevorzugten Ausführungsformen der Erfindung werden folglich temperaturstabilere Protease-Varianten bereitgestellt.

Unter Reinigungsleistung wird im Rahmen der Erfindung die Aufhellungsleistung an einer oder mehreren Anschmutzung(en), insbesondere auf Textilien, Wäsche oder Geschirr, verstanden. Im Rahmen der Erfindung weist sowohl das Wasch- und/oder Reinigungsmittel, welches die Protease umfasst bzw. die durch dieses Mittel gebildete Wasch- oder Reinigungsflotte, als auch die Protease selbst eine jeweilige Reinigungsleistung auf. Die Reinigungsleistung des Enzyms trägt somit zur Reinigungsleistung des Mittels bzw. der durch das Mittel gebildeten Wasch- oder Reinigungsflotte bei. Die Reinigungsleistung wird bevorzugt ermittelt wie weiter unten angegeben.

Unter Wasch- bzw. Reinigungsflotte wird diejenige das Wasch- oder Reinigungsmittel enthaltende Gebrauchslösung verstanden, die auf Textilien oder Gewebe bzw. harte Oberflächen, insbesondere Geschirr, einwirkt und damit mit den auf Textilien bzw. Geweben oder harten Oberflächen, insbesondere Geschirr, vorhandenen Anschmutzungen in Kontakt kommt. Üblicherweise entsteht die Wasch- bzw. Reinigungsflotte, wenn der Wasch- oder Reinigungsvorgang beginnt und das Wasch- oder Reinigungsmittel z.B. in einer Geschirrspülmaschine, einer Waschmaschine oder in einem anderen geeigneten Behältnis mit Wasser verdünnt wird.

Die Reinigungsleistung an Textilien oder Geweben kann in einem Waschsystem bestimmt werden, das ein Waschmittel in einer Dosierung zwischen 2,0 und 8,0 Gramm pro Liter Waschflotte enthält.

Die Konzentration des erfindungsgemäßen Peptids in dem für dieses Waschsystem bestimmten Waschmittel beträgt 1 x 10⁻⁸ bis 5 Gew.-%, vorzugsweise 0,0001 bis 1 Gew.-%, bevorzugt 0,0005 bis 0,5 Gew.-%, besonders bevorzugt 0,001 bis 0,1 Gew.-%, bezogen auf aktives Protein und Gesamtgewicht des Mittels.

Die Proteinkonzentration kann mit Hilfe bekannter Methoden, z.B. dem BCA-Verfahren (Bicinchoninsäure; 2,2'-Bichinolyl-4,4'-dicarbonsäure) oder dem Biuret-Verfahren (Gornall et al., J. Biol. Chem., 1948, 177, 751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (Bender et al., J. Am. Chem. Soc., 1966, 88, 24, 5890-5913) erfolgen.

Ein flüssiges Referenzwaschmittel für ein solches Waschsystem kann z.B. wie folgt zusammengesetzt sein (alle Angaben in Gew.-%): 4,4% Alkylbenzolsulfonsäure, 5,6% weitere anionische Tenside, 2,4% C₁₂₋₁₈ Na-Salze von Fettsäuren (Seifen), 4,4% nicht-ionische Tenside, 0,2% Phosphonate, 1,4% Zitronensäure, 0,95% NaOH, 0,01% Entschäumer, 2% Glycerin, 0,08% Konservierungsstoffe, 1% Ethanol, Rest demineralisiertes Wasser. Bevorzugt beträgt die Dosierung des flüssigen Waschmittels zwischen 3,0 und 6,0 Gramm pro Liter Waschflotte, z.B. 3,0, 3,2, 3,5, 3,7, 4,0, 4,5, 4,7, 4,9 oder 5,9 Gramm pro Liter Waschflotte. Bevorzugt wird gewaschen in einem pH-Wertebereich zwischen pH 7 und pH 10,5, bevorzugt zwischen pH 8 und pH 9.

Die Reinigungsleistung wird gegenüber einer Anschmutzung auf Textil durch Messung des Reinigungsgrades der gewaschenen Textilien bestimmt. Beispielsweise kann der Waschvorgang für 60 Minuten bei einer Temperatur von 40°C erfolgen und das Wasser eine Wasserhärte zwischen 15,5°dH und 16,5°dH (deutsche Härte) aufweisen.

Der Weißegrad, d.h. die Aufhellung der Anschmutzungen, als Maß für die Reinigungsleistung wird mit optischen Messverfahren bestimmt, bevorzugt photometrisch. Ein hierfür geeignetes Gerät ist z.B. das Spektrometer Minolta CM508d. Üblicherweise werden die für die Messung eingesetzten Geräte zuvor mit einem Weißstandard, bevorzugt einem mitgelieferten Weißstandard, kalibriert.

Durch den aktivitätsgleichen Einsatz der jeweiligen Enzyme wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also z.B. die Reinigungsleistung an bestimmten Anschmutzungen, verglichen werden. Generell gilt, dass eine niedrige spezifische Aktivität durch Zugabe einer größeren Proteinmenge ausgeglichen werden kann. Ferner können die zu untersuchenden Enzyme auch in gleicher Stoffmenge oder Gewichtsmenge eingesetzt werden, falls die zu untersuchenden Enzyme in einem Aktivitätstest eine unterschiedliche Affinität an das Testsubstrat aufweisen. Der Ausdruck "gleiche Stoffmenge" bezieht sich in diesem Zusammenhang auf eine molgleiche Verwendung der zu untersuchenden Enzyme. Der Ausdruck "gleiche Gewichtsmenge" bezieht sich auf einen gewichtsgleichen Einsatz der zu untersuchenden Enzyme.

Bevorzugte Ausführungsformen erfindungsgemäßer Verwendungen und Mittel erzielen solche vorteilhaften Reinigungsleistungen auch schon bei niedrigen Temperaturen, vorzugsweise in einem Temperaturbereich von etwa 10°C bis etwa 60°C, bevorzugt etwa 15°C bis etwa 40°C, besonders bevorzugt etwa 20°C bis etwa 30°C.

Die erfindungsgemäßen Proteasen weisen enzymatische Aktivität auf, d.h. sie sind zur Hydrolyse von Peptiden und Proteinen befähigt, insbesondere in einem Wasch- oder Reinigungsmittel, bevorzugt Textilwaschmittel. Eine erfindungsgemäße Protease ist daher ein Enzym, welches die Hydrolyse von Amid/Peptidbindungen in Protein/Peptid-Substraten katalysiert und dadurch in der Lage ist, Proteine oder Peptide zu spalten.

Verfahren zur Bestimmung der Protease-Aktivität dem Fachmann auf dem Gebiet der Enzymtechnologie geläufig und werden von ihm routinemäßig angewendet. Beispielsweise sind solche Verfahren offenbart in Tenside, Band 7 (1970), S. 125-132. Alternativ kann die Protease-Aktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20 s bis 60 s. Die Protease-Aktivität wird üblicherweise in Protease-Einheiten (PE) angegeben. Geeignete Protease-Aktivitäten betragen z.B. 2,25, 5 oder 10 PE pro ml Waschflotte. Die Protease-Aktivität ist jedoch nicht gleich Null.

Ein alternativer Test zur Feststellung der proteolytischen Aktivität der erfindungsgemäßen Proteasen ist ein optisches Messverfahren, bevorzugt ein photometrisches Verfahren. Der hierfür geeignete Test umfasst die Protease-abhängige Spaltung des Substratproteins Casein. Dieses wird durch die Protease in eine Vielzahl kleinerer Teilprodukte gespalten. Die Gesamtheit dieser Teilprodukte weist eine erhöhte Absorption bei 290 nm gegenüber nicht gespaltenem Casein auf, wobei diese erhöhte Absorption unter Verwendung eines Photometers ermittelt werden und somit ein Rückschluss auf die enzymatische Aktivität der Protease gezogen werden kann.

Ferner handelt es sich bei einer erfindungsgemäßen Protease vorzugsweise um eine reife (mature) Protease, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

In verschiedenen Ausführungsformen der Erfindung ist die Protease ein frei vorliegendes Enzym. Dies bedeutet, dass die Protease mit allen Komponenten eines Mittels direkt agieren kann und, falls es sich bei dem Mittel um ein Flüssigmittel handelt, dass die Protease direkt mit dem Lösungsmittel des Mittels (z.B. Wasser) in Kontakt steht. In anderen Ausführungsformen kann ein Mittel Proteasen enthalten, die einen Interaktionskomplex mit anderen Molekülen bilden oder die eine "Umhüllung" enthalten. Hierbei kann ein einzelnes oder mehrere Protease-Molekül(e) durch eine sie umgebende Struktur von den anderen Bestandteilen des Mittels getrennt sein. Eine solche trennende Struktur kann entstehen durch, ist allerdings nicht beschränkt auf, Vesikel, wie etwa eine Micelle oder ein Liposom. Die umgebende Struktur kann aber auch ein Viruspartikel, eine bakterielle Zelle oder eine eukaryotische Zelle sein. In verschiedenen Ausführungsformen kann ein Mittel Zellen von z.B. *Bacillus pumilus* oder *Bacillus subtilis* oder anderen Expressionsstämmen, die die erfindungsgemäßen Proteasen exprimieren, oder Zellkulturüberstände solcher Zellen enthalten.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. z.B. Altschul et al. (1990) Basic local alignment search tool, J. Mol. Biol., 215:403-410, und Altschul et al. (1997) Gapped BLAST and PSI-BLAST: a new generation of protein database search programs, Nucleic Acids Res., 25:3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden z.B. die Clustal-Serie (vgl. z.B. Chenna et al. (2003) Multiple sequence alignment with the Clustal series of programs, Nucleic Acid Res., 31:3497-3500), T-Coffee (vgl. z.B. Notredame et al. (2000) T-Coffee: A novel method for multiple sequence alignments, J. Mol. Biol., 302:205-217) oder Programme, die auf diesen Programmen bzw. Algorithmen basieren. Ferner möglich sind Sequenzvergleiche (Alignments) mit dem Computer-Programm Vector NTI^{®} Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert, oder Clone Manager 10 (Verwendung der Scoring Matrix BLOSUM 62 für Sequenz-Alignment auf Aminosäureebene). Soweit nicht anders angegeben, wird die hierin angegebene Sequenzidentität mit dem BLAST-Algorithmus bestimmt.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essenzielle Funktionen aus. Es kann daher sinnvoll sein, Sequenzübereinstimmungen nur auf einzelne, ggf. kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO:1 in einem wie oben definierten Alignment zugeordnet ist. Weiterhin richtet sich die Zuordnung der Positionen nach dem reifen (maturen) Protein. Diese Zuordnung ist insbesondere auch anzuwenden, wenn die Aminosäuresequenz einer erfindungsgemäßen Protease eine höhere Zahl von Aminosäureresten umfasst als die Protease gemäß SEQ ID NO:1. Ausgehend von den genannten Positionen in der Aminosäuresequenz der Protease gemäß SEQ ID NO:1 sind die Veränderungspositionen in einer erfindungsgemäßen Protease diejenigen, die eben diesen Positionen in einem Alignment zugeordnet sind.

Zusätzlich zu den vorstehend erläuterten Aminosäureveränderungen können erfindungsgemäße Proteasen weitere Aminosäureveränderungen, insbesondere Aminosäure-Substitutionen, -Insertionen oder -Deletionen, aufweisen. Solche Proteasen sind z.B. durch gezielte genetische Veränderung, d.h. durch Mutageneseverfahren, weiterentwickelt und für bestimmte Einsatzzwecke oder hinsichtlich spezieller Eigenschaften (z.B. hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Proteasen oder anderer Polypeptide genutzt werden. Das Ziel ist es, in die bekannten Moleküle gezielte Mutationen wie Substitutionen, Insertionen oder Deletionen einzuführen, um z.B. die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann z.B. die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität oder katalytische Aktivität der Protease erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Protease, z.B. hinsichtlich ihrer Stabilität bei Lagerung und/oder Aktivität und/oder ihrer Toleranz in Bezug auf das Substratspektrum, kann daher im Rahmen der Erfindung zusätzlich weiterentwickelt sein.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere oder alternative Austausche innerhalb derselben Polypeptidkette werden durch Schrägstriche voneinander getrennt. "130D/V" bedeutet somit, dass die Position 130 zu D oder V mutiert ist. Bei Insertionen sind nach der Sequenzposition zusätzliche Aminosäuren benannt. Bei Deletionen ist die fehlende Aminosäure durch ein Symbol, z.B. einen Stern oder einen Strich, ersetzt oder vor der entsprechenden Position ein Δ angegeben. Beispielsweise beschreibt P9T die Substitution von Prolin an Position 9 durch Threonin, P9TH die Insertion von Histidin nach der Aminosäure Threonin an Position 9 und P9* oder ΔP9 die Deletion von Prolin an Position 9. Diese Nomenklatur ist dem Fachmann auf dem Gebiet der Enzymtechnologie bekannt.

Ein weiterer Gegenstand der Erfindung ist daher eine Protease, die dadurch gekennzeichnet ist, dass sie aus einer Protease wie hierin beschrieben als Ausgangsmolekül erhältlich ist durch ein- oder mehrfache konservative Aminosäuresubstitution, wobei die Protease in der Zählung gemäß SEQ ID NO:1 mindestens eine der vorstehend beschriebenen Aminosäuresubstitutionen aufweist. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z.B. der Austausch eines unpolaren Aminosäurerestes gegen einen anderen unpolaren Aminosäurerest. Konservative Aminosäuresubstitutionen im Rahmen der Erfindung umfassen z.B.: G=A=S, I=V=L=M, D=E, N=Q, K=R, Y=F, S=T, G=A=I=V=L=M=Y=F=W=P=S=T.

Alternativ oder ergänzend ist die Protease dadurch gekennzeichnet, dass sie aus einer erfindungsgemäßen Protease als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 255, 256, 257, 258, 259, 260, 261, 262, 263, 264, 265, 266, 267, 268 oder 269 zusammenhängenden Aminosäuren mit dem Ausgangsmolekül übereinstimmt, wobei die Protease, jeweils bezogen auf die Nummerierung gemäß SEQ ID NO:1, an mindestens einer der Positionen, die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist.

So ist es z.B. möglich, an den Termini oder in den Loops des Enzyms einzelne Aminosäuren zu deletieren, ohne dass dadurch die proteolytische Aktivität verloren oder vermindert wird. Ferner kann durch derartige Fragmentierung, Deletions-, Insertions- oder Substitutionsmutagenese z.B. auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms, d.h. in einer bevorzugten Ausführungsform beträgt die proteolytische Aktivität mindestens 100%, vorzugsweise mindestens 105%, weiter bevorzugt mindeste 110%, noch weiter bevorzugt mindestes 120% oder mehr, der Aktivität des Ausgangsenzyms. Auch weitere Substitutionen können vorteilhafte Wirkungen zeigen. Sowohl einzelne wie auch mehrere zusammenhängende Aminosäuren können gegen andere Aminosäuren ausgetauscht werden.

Vorteilhafte Positionen für Sequenzveränderungen, insbesondere Substitutionen, der Protease gemäß SEQ ID NO:1, die übertragen auf homologe Positionen der erfindungsgemäßen Proteasen bevorzugt von Bedeutung sind und der Protease vorteilhafte funktionelle Eigenschaften verleihen, sind demnach die Positionen, die in einem Alignment den hierin beschriebenen Positionen entsprechen, d.h. in der Zählung gemäß SEQ ID NO:1. An den genannten Positionen liegen in dem Wildtypmolekül der Protease folgende Aminosäurereste: 121N, 194A, 209A, 218T, 237N.

Eine weitere Bestätigung der korrekten Zuordnung der zu verändernden Aminosäuren, d.h. insbesondere deren funktionelle Entsprechung, können Vergleichsversuche liefern, wonach die beiden auf der Basis eines Alignments einander zugeordneten Positionen in beiden miteinander verglichenen Proteasen auf die gleiche Weise verändert werden und beobachtet wird, ob bei beiden die enzymatische Aktivität auf gleiche Weise verändert wird. Geht z.B. ein Aminosäureaustausch in einer bestimmten Position der Protease gemäß SEQ ID NO:1 mit einer Veränderung eines enzymatischen Parameters einher, z.B. mit der Erhöhung des K_{M}-Wertes, und wird eine entsprechende Veränderung des enzymatischen Parameters, z.B. also ebenfalls eine Erhöhung des K_{M}-Wertes, in einer erfindungsgemäßen Protease-Variante beobachtet, deren Aminosäureaustausch durch dieselbe eingeführte Aminosäure erreicht wurde, so ist hierin eine Bestätigung der korrekten Zuordnung zu sehen.

Alle genannten Sachverhalte sind auch auf die erfindungsgemäßen Verfahren zur Herstellung einer Protease anwendbar.

Ein erfindungsgemäßes Verfahren zur Herstellung einer Protease, umfasst das Einbringen mindestens einer Aminosäuresubstitution an mindestens einer der Positionen, die bezogen auf die Nummerierung gemäß SEQ ID NO:1 den Positionen 121, 194, 209, 218 und 237 entsprechen, ausgewählt aus der N121F, A194C, A209V, T218I, N237P und N237W bestehenden Gruppe, in ein Ausgangsmolekül, das eine Aminosäuresequenz aufweist, die mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% oder 100% Sequenzidentität mit der in SEQ ID NO:1 angegebenen Aminosäuresequenz über deren Gesamtlänge aufweist.

Ein erfindungsgemäßes Verfahren kann ferner einen oder mehrere der folgenden Verfahrensschritte umfassen:
(a) Einbringen einer ein- oder mehrfachen konservativen Aminosäuresubstitution, wobei die Protease, jeweils bezogen auf die Nummerierung gemäß SEQ ID NO:1, an mindestens einer der Positionen, die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist;
(b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 190, 200, 210, 220, 230, 240, 250, 255, 256, 257, 258, 259, 260, 261,262,263, 264, 265, 266, 267, 268 oder 269 zusammenhängenden Aminosäuren mit dem Ausgangsmolekül übereinstimmt, wobei die Protease, jeweils bezogen auf die Nummerierung gemäß SEQ ID NO:1, an mindestens einer der Positionen, die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist.

Sämtliche Ausführungsformen gelten auch für die erfindungsgemäßen Verfahren.

In einer weiteren Ausführungsform der Erfindung ist eine zuvor beschriebene Protease weiter stabilisiert. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Beispiele für hierfür geeignete Sequenzveränderungen sind vorstehend genannt. Weitere geeignete Sequenzveränderungen sind aus dem Stand der Technik bekannt.

Weitere Möglichkeiten der Stabilisierung sind z.B.:
- Veränderung der Bindung von Metallionen, insbesondere der Calcium-Bindungsstellen, z.B. durch Austauschen von einer oder mehreren der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Alternativ oder ergänzend kann die erfindungsgemäße Protease mit mindestens einer reversiblen Inhibitorverbindung, die aus der aus Peptidinhibitoren, insbesondere Peptidaldehyde, Polyolen, insbesondere Glycerin und 1,2-Propylenglykol, Benzamidinhydrochlorid, Borax, Borsäuren, Boronsäuren oder deren Salze oder Ester oder Derivate, insbesondere Phenylboronsäurederivate oder 4-Formylphenylboronsäure (4-FPBA) kombiniert werden, um die Stabilität der Protease in Wasch- und Reinigungsmitteln weiter zu erhöhen. Besonders bevorzugte reversible Proteaseinibitoren umfassen Borsäure, 4-FPBA und Peptidinhibitoren.

In verschiedenen Ausführungsformen können das Enzym und die Inhibitorverbindung in einer Enzymzusammensetzung vorformuliert vorliegen. Bevorzugt eingesetzte Protease-Zubereitungen enthalten zwischen 0,1 und 40 Gew.-%, bevorzugt zwischen 0,2 und 30 Gew.-%, besonders bevorzugt zwischen 0,4 und 20 Gew.-% und insbesondere zwischen 0,8 und 10 Gew.-% des Enzymproteins. In solchen Zusammensetzungen kann die Inhibitorverbindung in einer Menge von 0,05 bis 35 Gew.-%, vorzugsweise von 0,05 bis 10 Gew.-%, bezogen auf das Gesamtgewicht in der Enzymzusammensetzung, enthalten sein. Diese Enzymzusammensetzung, die ebenfalls ein Bestandteil der vorliegenden Erfindung ist, kann dann in Wasch- oder Reinigungsmitteln eingesetzt werden, und zwar in Mengen, die zu den gewünschten Endkonzentrationen im Wasch- oder Reinigungsmittel führen.

Ein weiterer Gegenstand der Erfindung ist eine Protease wie hierin beschrieben, die dadurch gekennzeichnet ist, dass sie mindestens eine chemische Modifikation aufweist. Eine Protease mit einer solchen Veränderung wird als Derivat bezeichnet, d.h. die Protease ist derivatisiert. Unter Derivaten werden im Sinne der vorliegenden Anmeldung demnach solche Proteine verstanden, deren reine Aminosäurekette chemisch modifiziert worden ist. Solche Derivatisierungen können z.B. *in vivo* durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch *in vitro* durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das z.B. über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können z.B. die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann z.B. für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit z.B. dessen Hautverträglichkeit zu erhöhen. Beispielsweise können Kopplungen mit makromolekularen Verbindungen, z.B. Polyethylenglykol, das Protein hinsichtlich der Stabilität und/oder Hautverträglichkeit verbessern. Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen kombiniert sein, z.B. aus der Kultur der produzierenden Mikroorganismen. Ein Protein kann auch, z.B. zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann z.B. über entsprechende Begleitstoffe gesteuert werden. Insbesondere die gemeinsame Präparation von Proteasen mit spezifischen Inhibitoren ist diesbezüglich möglich. Unter allen hierin beschriebenen Proteasen bzw. Protease-Varianten und/oder -Derivaten sind im Rahmen der vorliegenden Erfindung diejenigen besonders bevorzugt, deren Lagerstabilität und/oder katalytische Aktivität und/oder Substrattoleranz und/oder Reinigungsleistung gegenüber der Ausgangsvariante verbessert ist, wobei die katalytische Aktivität und/oder Reinigungsleistung wie hierin beschrieben bestimmt wird.

Ein weiterer Gegenstand der Erfindung ist eine Nukleinsäure, die für eine erfindungsgemäße Protease codiert, sowie ein Vektor enthaltend eine solche Nukleinsäure, insbesondere ein Klonierungsvektor oder ein Expressionsvektor. Hierbei kann es sich um DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren codieren können, so dass eine bestimmte Aminosäuresequenz von mehreren unterschiedlichen Nukleinsäuren codiert werden kann. Auf Grund dieser Degeneriertheit des genetischen Codes sind sämtliche Nukleinsäuresequenzen in diesem Erfindungsgegenstand eingeschlossen, die eine der vorstehend beschriebenen Proteasen codieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz codierende Nukleinsäuren problemlos ermitteln. Weiterhin können bei erfindungsgemäßen Nukleinsäuren ein oder mehrere Codons durch synonyme Codons ersetzt sein. Dieser Aspekt bezieht sich insbesondere auf die heterologe Expression der erfindungsgemäßen Enzyme. So besitzt jeder Organismus, z.B. eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure codierend führt das dazu, dass in dem Organismus ein Codon weniger effizient translatiert wird als ein synonymes Codon, das für dieselbe Aminosäure codiert. Auf Grund des Vorliegens einer höheren Anzahl von tRNA-Molekülen für das synonyme Codon kann dieses in dem Organismus effizienter translatiert werden.

Einem Fachmann ist es über heutzutage allgemein bekannte Methoden, wie z.B. die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind z.B. aus Sambrook, J., Fritsch, E.F. and Maniatis, T. 2001. Molecular cloning: a laboratory manual, 3. Edition Cold Spring Laboratory Press bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zelllinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen z.B. solche, deren Ursprung bakterielle Plasmide, Viren oder Bakteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren. Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, z.B. durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht-menschliche Wirtszelle, die eine erfindungsgemäße Nukleinsäure oder einen erfindungsgemäßen Vektor beinhaltet, oder die eine erfindungsgemäße Protease beinhaltet, insbesondere eine, die die Protease in das die Wirtszelle umgebende Medium sezerniert. Bevorzugt wird eine erfindungsgemäße Nukleinsäure oder ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile oder -Fragmente einer erfindungsgemäßen Nukleinsäure derart in eine Wirtszelle eingebracht werden, dass die dann resultierende Wirtszelle eine erfindungsgemäße Nukleinsäure oder einen erfindungsgemäßen Vektor enthält. Dieses Vorgehen eignet sich besonders dann, wenn die Wirtszelle bereits einen oder mehrere Bestandteile einer erfindungsgemäßen Nukleinsäure oder eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was z.B. die Transformation mit der Nukleinsäure oder dem Vektor und dessen stabile Etablierung angeht, z.B. einzellige Pilze oder Bakterien. Ferner zeichnen sich bevorzugte Wirtszellen durch eine gute mikrobiologische und biotechnologische Handhabbarkeit aus. Das betrifft z.B. leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Proteasen von den sie produzierenden Zellen nach deren Herstellung modifiziert werden, z.B. durch Anknüpfung von Zuckermolekülen, Formylierungen, Aminierungen, usw. Solche posttranslationalen Modifikationen können die Protease funktionell beeinflussen.

Weitere bevorzugte Ausführungsformen stellen solche Wirtszellen dar, die aufgrund genetischer Regulationselemente, die z.B. auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryotische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein. Bei gramnegativen Bakterien wie z.B. *Escherichia coli* wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie z.B. Bacilli oder Actinomyceten oder andere Vertreter der *Actinomycetales* besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist. Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren. Die vorliegende Erfindung ist prinzipiell auf alle Mikroorganismen, insbesondere auf alle fermentierbaren Mikroorganismen, besonders bevorzugt auf solche der Gattung *Bacillus,* anwendbar und führt dazu, dass sich durch den Einsatz solcher Mikroorganismen erfindungsgemäße Proteine herstellen lassen. Solche Mikroorganismen stellen dann Wirtszellen im Sinne der Erfindung dar. In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von *Escherichia, Klebsiella, Bacillus, Staphylococcus, Corynebacterium, Arthrobacter, Streptomyces, Stenotrophomonas* und *Pseudomonas,* weiter bevorzugt eines, das ausgewählt ist aus der Gruppe von *Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor* und *Stenotrophomonas maltophilia.*

Die Wirtszelle kann aber auch eine eukaryotische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt. Im Gegensatz zu prokaryotischen Zellen sind eukaryotische Zellen in der Lage, das gebildete Protein posttranslational zu modifizieren. Beispiele dafür sind Pilze wie Actinomyceten oder Hefen wie *Saccharomyces* oder *Kluyveromyces.* Dies kann z.B. dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryotische Systeme besonders im Zusammenhang mit der Proteinsynthese durchführen, gehören z.B. die Bindung niedermolekularer Verbindungen wie Membrananker oder Oligosaccharide. Derartige Oligosaccharid-Modifikationen können z.B. zur Senkung der Allergenizität eines exprimierten Proteins wünschenswert sein. Auch eine Co-Expression mit den natürlicherweise von derartigen Zellen gebildeten Enzymen, wie z.B. Cellulasen, kann vorteilhaft sein. Ferner können sich z.B. thermophile pilzliche Expressionssysteme besonders zur Expression temperaturbeständiger Proteine oder Varianten eignen.

Die erfindungsgemäßen Wirtszellen werden in üblicher Weise kultiviert und fermentiert, z.B. in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Wirtszellen werden bevorzugt verwendet, um erfindungsgemäße Proteasen herzustellen. Ein weiterer Gegenstand der Erfindung ist daher ein Verfahren zur Herstellung einer Protease umfassend
a) Kultivieren einer erfindungsgemäßen Wirtszelle, und
b) Isolieren der Protease aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, z.B. der erfindungsgemäßen Proteasen. Alle Fermentationsverfahren, die auf einem entsprechenden Verfahren zur Herstellung einer erfindungsgemäßen Protease beruhen, stellen Ausführungsformen dieses Erfindungsgegenstandes dar. Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse bzw. Trockenmasse und/oder insbesondere in der Aktivität der interessierenden Protease erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden. Die hergestellte Protease kann aus dem Fermentationsmedium geerntet werden. Ein solches Fermentationsverfahren ist gegenüber einer Isolation der Protease aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern oder -mechanismen und/oder Transportsystemen, damit die Wirtszellen die Protease in das Fermentationsmedium sezernieren. Ohne Sekretion kann alternativ die Isolation der Protease aus der Wirtszelle, d.h. eine Aufreinigung derselben aus der Zellmasse, erfolgen, z.B. durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Protease herzustellen.

Ein weiterer Gegenstand der Erfindung ist ein Wasch- und/oder Reinigungsmittel, das dadurch gekennzeichnet ist, dass es eine erfindungsgemäße Protease wie hierin beschrieben enthält. Bevorzugt ist das Mittel ein Textilwaschmittel, insbesondere ein flüssiges Textilwaschmittel.

In bevorzugten Ausführungsformen wird die erfindungsgemäße Protease in Mitteln eingesetzt, die im Wesentlichen frei von borhaltigen Verbindungen sind. "Im Wesentlichen frei von borhaltigen Verbindungen" bedeutet in diesem Zusammenhang, dass die entsprechenden Mittel weniger als 2 Gew.-%, vorzugsweise weniger als 1 Gew.-%, weiter bevorzugt weniger als 0,5 Gew.-% und besonders bevorzugt weniger als 0,1 Gew.-%, borhaltige Verbindungen, bezogen auf das Gesamtgewicht des Mittels, enthalten. In ganz besonders bevorzugten Ausführungsformen sind solche Mittel frei von borhaltigen Verbindungen, d.h. sie enthalten insbesondere keine Borsäure und/oder 4-FPBA.

In bevorzugten Ausführungsformen wird die erfindungsgemäße Protease in Mitteln eingesetzt, die im Wesentlichen frei von phosphonathaltigen Verbindungen sind. "Im Wesentlichen frei von phosphonathaltigen Verbindungen" bedeutet in diesem Zusammenhang, dass die entsprechenden Mittel weniger als 2 Gew.-%, vorzugsweise weniger als 1 Gew.-%, weiter bevorzugt weniger als 0,5 Gew.-% und besonders bevorzugt weniger als 0,1 Gew.-%, phosphonathaltige Verbindungen, bezogen auf das Gesamtgewicht des Mittels, enthalten. In besonders bevorzugten Ausführungsformen sind diese Mittel frei von phosphonathaltigen Verbindungen.

In bevorzugten Ausführungsformen wird die erfindungsgemäße Protease in Mitteln eingesetzt, die im Wesentlichen frei von phosphathaltigen Verbindungen sind. "Im Wesentlichen frei von phosphathaltigen Verbindungen" bedeutet in diesem Zusammenhang, dass die entsprechenden Mittel weniger als 2 Gew.-%, vorzugsweise weniger als 1 Gew.-%, weiter bevorzugt weniger als 0,5 Gew.-% und besonders bevorzugt weniger als 0,1 Gew.-%, phosphathaltige Verbindungen, bezogen auf das Gesamtgewicht des Mittels, enthalten. In besonders bevorzugten Ausführungsformen sind diese Mittel frei von phosphathaltigen Verbindungen.

Unter einem Wasch- oder Reinigungsmittel sind erfindungsgemäß alle denkbaren Wasch- oder Reinigungsmittelarten zu verstehen, sowohl Konzentrate als auch unverdünnt anzuwendende Mittel, zum Einsatz im kommerziellen Maßstab, in der Waschmaschine oder bei der Handwäsche bzw. -reinigung. Dazu gehören z.B. Waschmittel für Textilien, Teppiche, oder Naturfasern, für die die Bezeichnung Waschmittel verwendet wird. Dazu gehören z.B. auch Geschirrspülmittel für Geschirrspülmaschinen (maschinelle Geschirrspülmittel) oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln z.B. auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, z.B. zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet. Mit umfasst sind dabei auch Mittel zur Verwendung in (halb-)automatisierten Wasch- oder Reinigungssystemen wie z.B. Wischrobotor oder Nassstaubsauger.

Erfindungsgemäße Mittel, die als pulverförmige oder granulare Feststoffe, in verdichteter oder nachverdichteter Teilchenform, als homogene Lösungen oder Suspensionen vorliegen können, können neben einer erfindungsgemäßen Protease alle bekannten und in derartigen Mitteln üblichen Inhaltsstoffe enthalten, wobei bevorzugt mindestens ein weiterer Inhaltsstoff in dem Mittel vorhanden ist. Erfindungsgemäße Mittel können insbesondere Tenside, Builder (Gerüststoffe), Polymere, Glaskorrosionsinhibitoren, Korrosionsinhibitoren, Bleichmittel wie Persauerstoffverbindungen, Bleichaktivatoren oder Bleichkatalysatoren enthalten. Ferner können sie wassermischbare organische Lösungsmittel, weitere Enzyme, Enzymstabilisatoren, Sequestrierungsmittel, Elektrolyte, pH-Regulatoren und/oder weitere Hilfsstoffe wie optische Aufheller, Vergrauungsinhibitoren, Farbübertragungsinhibitoren, Schaumregulatoren sowie Farb- und Duftstoffe sowie Kombinationen hiervon enthalten. Vorteilhafte Inhaltsstoffe erfindungsgemäßer Mittel sind offenbart in WO 2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende Patentanmeldung einbezogen.

Erfindungsgemäße Mittel enthalten die erfindungsgemäße Protease vorteilhafterweise in einer Menge von 2 µg bis 20 mg, vorzugsweise von 5 µg bis 17,5 mg, besonders bevorzugt von 20 µg bis 15 mg und ganz besonders bevorzugt von 50 µg bis 10 mg pro g des Mittels. In verschiedenen Ausführungsformen beträgt die Konzentration der hierin beschriebenen Protease (aktives Enzym) in dem Mittel >0 bis 1 Gew.-%, vorzugsweise 0,0001 oder 0,001 bis 0,1 Gew.-% bezogen auf das Gesamtgewicht des Mittels.

Erfindungsgemäße Mittel enthalten die erfindungsgemäße Protease zunehmend bevorzugt in einer Menge von 1 x 10⁻⁸ bis 5 Gew.-%, von 0,0001 bis 1 Gew.-%, von 0,0005 bis 0,5 Gew.-%, von 0,001 bis 0,1 Gew.-%, jeweils bezogen auf aktives Protein und bezogen auf das Gesamtgewicht des Mittels.

Weitere Ausführungsformen umfassen alle festen, pulverförmigen, flüssigen, gelförmigen oder pastösen Darreichungsformen erfindungsgemäßer Mittel, die ggf. auch aus mehreren Phasen bestehen können sowie in komprimierter oder nicht komprimierter Form vorliegen können. Erfindungsgemäße Mittel können als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen erfindungsgemäßer Mittel zählen ferner Extrudate, Granulate, Tabletten oder Pouches. Alternativ kann erfindungsgemäße Mittel auch flüssig, gelförmig oder pastös sein, z.B. in Form eines nicht-wässrigen Flüssigwaschmittels oder einer nicht-wässrigen Paste oder in Form eines wässrigen Flüssigwaschmittels oder einer wasserhaltigen Paste. Flüssige Mittel sind generell bevorzugt. Weiterhin können erfindungsgemäße Mittel als Einkomponentensystem vorliegen. Solche Mittel bestehen aus einer Phase. Alternativ können erfindungsgemäße Mittel auch aus mehreren Phasen bestehen. Ein solches Mittel ist demnach in mehrere Komponenten aufgeteilt.

In einer bevorzugten Ausführungsform ist das erfindungsgemäße Mittel ein Textilwaschmittel.

In einer bevorzugten Ausführungsform ist das erfindungsgemäße Mittel ein flüssiges Textilwaschmittel.

In einer bevorzugten Ausführungsform ist das erfindungsgemäße Mittel ein vorportioniertes Waschmittel, insbesondere eine Waschmittelportionseinheit umfassend eine erfindungsgemäße Waschmittelzubereitung und einen wasserlöslichen Film, welcher die Waschmittelzubereitung vollständig umschließt.

Der wasserlösliche Film, in welche die Waschmittelzubereitung verpackt ist, kann ein oder mehrere strukturell verschiedene wasserlösliche(s) Polymer(e) umfassen. Als wasserlösliche(s) Polymer(e) eignen sich insbesondere Polymere aus der Gruppe (ggf. acetalisierter) Polyvinylalkohole (PVAL) sowie deren Copolymere. Geeignete wasserlösliche Filme zum Einsatz werden u.a. von der Firma MonoSol LLC z.B. unter der Bezeichnung M8630, M8720, M8310, C8400 oder M8900 vertrieben. Geeignet sind z.B. auch Filme mit der Bezeichnung Solublon^{®} PT, Solublon^{®} GA, Solublon^{®} KC oder Solublon^{®} KL von der Aicello Chemical Europe GmbH oder die Folien VF-HP von Kuraray.

Wenn erfindungsgemäße Mittel in flüssiger Form vorliegen, enthalten sie vorzugsweise bezogen auf ihr Gesamtgewicht mehr als 40 Gew.-%, vorzugsweise 50 bis 90 Gew.-% und besonders bevorzugt 60 bis 80 Gew.-% Wasser.

Erfindungsgemäße Mittel können ausschließlich eine erfindungsgemäße Protease enthalten. Alternativ können sie auch weitere hydrolytische Enzyme oder andere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Lipase, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder andere - von den erfindungsgemäßen Proteasen unterscheidbare - Protease, sowie deren Gemische. Weitere Enzyme sind in dem Mittel vorteilhafterweise jeweils in einer Menge von 1 x 10⁻⁸ bis 5 Gew.-% bezogen auf aktives Protein enthalten. Zunehmend bevorzugt ist jedes weitere Enzym in einer Menge von 1 x 10⁻⁷ bis 3 Gew.-%, von 0,00001 bis 1 Gew.-%, von 0,00005 bis 0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in dem Mittel enthaltenen Enzyme unterstützen sich in ihrer Reinigungsleistung gegenseitig. Ganz besonders bevorzugt liegt ein solcher Synergismus vor zwischen der erfindungsgemäß enthaltenen Protease und einem weiteren Enzym eines erfindungsgemäßen Mittels. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Erfindungsgemäß bevorzugte Textilwaschmittel weisen mindestens eine Protease und mindestens eine Amylase auf. In einer weiteren bevorzugten Ausführungsform der Erfindung weisen Textilwaschmittel mindestens eine Protease und mindestens eine Cellulase auf. In einer weiteren bevorzugten Ausführungsform weisen Textilwaschmittel mindestens eine Protease und mindestens eine Lipase auf. In einer weiteren bevorzugten Ausführungsform weisen Textilwaschmittel mindestens eine Protease, mindestens eine Amylase und mindestens eine Lipase auf. In einer weiteren bevorzugten Ausführungsform weisen Textilwaschmittel mindestens eine Protease, mindestens eine Amylase und mindestens eine Cellulase auf. In einer weiteren bevorzugten Ausführungsform weisen Textilwaschmittel mindestens eine Protease, mindestens eine Amylase, mindestens eine Cellulase und mindestens eine Lipase auf. Besonders bevorzugt sind Textilwaschmittel, welche 3 bis 10 verschiedene Enzyme aufweisen, wobei Textilwaschmittel, welche 3 bis 10 unterschiedliche Enzymarten aufweisen, im Hinblick auf die Reinigungsleistung gegenüber einem sehr großen Fleckenspektrum von besonderer Bevorzugung sein können.

In den hierin beschriebenen Reinigungsmitteln können die einzusetzenden Enzyme ferner zusammen mit Begleitstoffen, etwa aus der Fermentation, konfektioniert sein. In flüssigen Formulierungen werden die Enzyme bevorzugt als Enzymflüssigformulierung(en) eingesetzt.

Die Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen z.B. die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, z.B. durch Sprühtrocknung oder Extrusion der Enzymlösung zusammen mit einem vorzugsweise natürlichen Polymer oder in Form von Kapseln, z.B. solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, z.B. Stabilisatoren, Emulgatoren, Pigmente, Bleich- oder Farbstoffe aufgebracht werden. Derartige Kapseln werden nach an sich bekannten Methoden, z.B. durch Schüttel- oder Rollgranulation oder in Fluid-bed-Prozessen aufgebracht. Vorteilhafterweise sind derartige Granulate, z.B. durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Die Enzyme können auch in wasserlösliche Filme, wie sie z.B. bei der Konfektionierung von Wasch- und Reinigungsmitteln in Einheitsdosisform verwendet werden, eingebracht werden. Ein derartiger Film ermöglicht die Freisetzung der Enzyme nach Kontakt mit Wasser. Wie hierin verwendet, bezieht sich "wasserlöslich" auf eine Filmstruktur, die vorzugsweise vollständig wasserlöslich ist. Bevorzugt besteht ein solcher Film aus (vollständig oder teilweise hydrolysiertem) Polyvinylalkohol (PVA).

Ein weiterer Erfindungsgegenstand ist ein Verfahren zur Reinigung von Textilien und/oder harten Oberflächen, insbesondere Geschirr, das dadurch gekennzeichnet ist, dass in mindestens einem Verfahrensschritt ein Mittel mit einer erfindungsgemäßen Protease angewendet wird. In verschiedenen Ausführungsformen zeichnet sich das beschriebene Verfahren dadurch aus, dass die Protease bei einer Temperatur von etwa 0°C bis etwa 100°C, bevorzugt etwa 20°C bis etwa 60°C und weiter bevorzugt etwa 20°C bis etwa 40°C eingesetzt wird.

Hierunter fallen sowohl manuelle als auch maschinelle Verfahren, wobei maschinelle Verfahren aufgrund ihrer präziseren Steuerbarkeit, was z.B. die eingesetzten Mengen und Einwirkzeiten angeht, bevorzugt sind. Verfahren zur Reinigung von Textilien zeichnen sich im Allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird.

Da erfindungsgemäße Proteasen natürlicherweise bereits eine hydrolytische Aktivität besitzen und diese auch in Medien entfalten, die sonst keine Reinigungskraft besitzen wie z.B. in bloßem Puffer, kann ein einzelner und/oder der einzige Schritt eines solchen Verfahrens darin bestehen, dass als einzige reinigungsaktive Komponente eine erfindungsgemäße Protease mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Protease aktiv wird. Hierunter sind Verfahren für Textilrohstoffe, Fasern oder Textilien mit natürlichen Bestandteilen bevorzugt, und ganz besonders für solche mit Wolle oder Seide.

Ein weiterer Gegenstand der Erfindung betrifft ein Verfahren zur Entfernung von proteasesensitiven Anschmutzungen, insbesondere ei(gelb)-haltigen Anschmutzungen, von Textilien und/oder harten Oberflächen, insbesondere Geschirr, wobei in mindestens einem Verfahrensschritt ein Mittel, das eine erfindungsgemäße Protease, wie hierin beschrieben, enthält und/oder eine erfindungsgemäße Protease, wie hierin beschrieben, angewendet wird.

Schließlich erfasst die Erfindung auch die Verwendung der hierin beschriebenen Proteasen in Wasch- oder Reinigungsmitteln, z.B. wie oben beschrieben, zur (verbesserten) Entfernung von peptid- oder proteinhaltigen Anschmutzungen, z.B. von Textilien und/oder harten Oberflächen.

In einer weiteren bevorzugten Ausführungsform betrifft die Erfindung die Verwendung einer hierin beschriebenen erfindungsgemäßen Protease in einem Wasch- oder Reinigungsmittel, insbesondere Textilwaschmittel, zur Verbesserung der Lagerstabilität einer Protease in einem solchen proteasehaltigen Wasch- oder Reinigungsmittel, insbesondere Textilwaschmittel.

In einer weiteren bevorzugten Ausführungsform betrifft die Erfindung die Verwendung einer hierin beschriebenen erfindungsgemäßen Protease in einem Wasch- oder Reinigungsmittel, insbesondere Textilwaschmittel, zur Entfernung mindestens einer proteasesensitiven Anschmutzung.

Alle Sachverhalte, Gegenstände und Ausführungsformen, die für die erfindungsgemäße Protease und sie enthaltende Mittel beschrieben sind, sind auch auf diese Erfindungsgegenstände anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren und Verwendungen gilt.

### BEISPIELE

**Tabelle1: Verwendete Reinigungsmittelmatrix**

| **Chemischer Name** | **Gew.-% Aktivsubstanz in Formulierung** |
|---|---|
| Alkylbenzolsulfonsäure | 22 |
| Nichtionische Tenside | 25 |
| C12-14 Fettsäure | 7 |
| 1,2-Propandiol | 4 |
| Glycerin | 10 |
| Monoethanolamin | 6 |
| Phosphonat | 1 |
| Sonstiges (reinigungsaktive Polymere, Konservierungsmittel, Parfüm, etc.) | Minors |
| Wasser | Ad 100 |
| Ohne Enzyme, pH 7,5 | |

**Tabelle 2: Verwendete Proteasen:**

| | **Aminosäuresubstitutionen gegenüber SEQ ID NO:1, Zählung gemäß SEQ ID NO:1** |
|---|---|
| Protease 1 (P1) | Protease gemäß SEQ ID NO:1 (Wildtyp) |
| Protease 2 (P2) | A209V-N237P |
| Protease 3 (P3) | A209V |
| Protease 4 (P4) | N121F-A209V-N237P |
| Protease 5 (P5) | A194C-T2181-N237W |
| Protease 6 (P6) | A209V-N237W |
| Protease 7 (P7) | A209V-N2181-N237P |

### Beispiel 1: Bestimmung der Lagerstabilität

Die Lagerstabilität der Proteasen gemäß Tabelle 2 wurden in einer Waschmittelzusammensetzung gemäß Tabelle 1 mit Bacillus subtilis Kulturüberständen, welche die zu untersuchende Protease enthielt, bestimmt. Die Kulturüberstände wurden im Verhältnis 1:5 mit einer Waschmittellösung (1:1 Waschmittelmatrix und entionisiertes Wasser) verdünnt. Die Waschmittellösung mit der zu untersuchenden Protease wurde bei einer Temperatur von 30°C für 2 Wochen gelagert.

Die Aktivität der zu untersuchenden Protease wurde unmittelbar nach Ansetzen der Versuchsansätze sowie nach 2 Wochen Lagerung gemessen.

### Protease-Aktivitätsassay

Die Aktivität der Protease wird durch die Freisetzung des Chromophors para-Nitroanilin aus dem Substrat Succinyl Alanin-Alanin-Prolin-Phenylalanin-para-Nitroanilid (AAPF-pNA; Bachem L-1400) bestimmt. Die Freisetzung des pNA verursacht eine Zunahme der Extinktion bei 410 nm, deren zeitlicher Verlauf ein Maß für die enzymatische Aktivität ist.

Die Messung erfolgte bei einer Temperatur von 30°C, bei pH 8,6 und einer Wellenlänge von 410 nm. Die Messzeit betrug 5 min bei einem Messintervall von 20 bis 60 Sekunden.

Messansatz:
10 µL AAPF-Lösung (70 mg/mL)
1000 µL Tris/HCl (0,1 M; pH 8,6 mit 0,1% Brij 35)
10 µL verdünnte Proteaselösung
Kinetik erstellt über 5 min bei 25°C (410 nm)

Die Aktivität der Protease vor Lagerung wurde auf 100% normiert. Im Folgenden sind die Differenzwerte der Restaktivität (RA) der Protease des jeweiligen Ansatzes nach Lagerung im Vergleich Anfangswert vor Lagerung angegeben. Eine Differenz ≥5% wird als signifikant angesehen.

| Name | RA (14d) | |
|---|---|---|
| | Mittelwert | Stabwn (n>2) |
| Protease 1 (P1) WT | 4% | 1,5% |
| Protease 1 (P2) | 12% | 1,0% |
| Protease 1 (P3) | 10% | 1,2% |
| Protease 1 (P4) | 38% | 0,0% |
| Protease 1 (P5) | 16% | 2,0% |
| Protease 1 (P6) | 10% | 1,7% |
| Protease 1 (P7) | 40% | 1,0% |

### Beispiel 2: Beispielformulierungen

Die erfindungsgemäßen Proteasen können in verschiedenen Wasch- und Reinigungsmittelzusammensetzungen eingesetzt werden und ihren Effekt verwirklichen.

**Tabelle 3: Flüssigwaschmittel**

| **Chemischer Name** | **Gew.-% Aktivsubstanz in der Formulierung** | | | | | |
|---|---|---|---|---|---|---|
| | **A** | **B** | **C** | **D** | **E** | **F** |
| Wasser demin. | Rest | Rest | Rest | Rest | Rest | Rest |
| LAS | 5,5 | 20 | 15,0 | 5,5 | 21,7 | 23,5 |
| FAEOS | 7,0 | | 5,0 | | | |
| Palmkernölsäure | 3,0 | 8,0 | | | 7,0 | 7,4 |
| FAEO | 5,5 | | 8,0 | | | |
| C_{13/15} Oxoalkohol, 8EO | | 25 | | | | |
| C₁₂₋₁₈ Fettalkoholethoxylat, 7EO | | | | | 22,4 | 23,4 |
| Alkylpolyglykosid | | | 4,0 | | | |
| Nichtionische Tenside | | | | 3,1 | | |
| Seife | | | 1,0 | 0,5 | | |
| HEDP | 0,5 | | | | | |
| DTPMPA 7Na | | 1,0 | 1,0 | 0,2 | 0,5 | 1,7 |
| Zitronensäure | 2,5 | | 3,0 | 0,23 | | |
| NaOH | 3,0 | | | 0,7 | | |
| Glycerin | 3,0 | 5,0 | | 0,5 | 9,4 | 10,2 |
| Ethanol | 1,5 | 3,0 | | | | 3,2 |
| 1,2-Propandiol | | 10,0 | 12,0 | | 5,0 | 5,6 |
| Monoethanolamin | | 6,0 | 7,0 | | 6,0 | 6,1 |
| Borsäure | 1,0 | | 1,0 | 0,5 | | |
| Polyalkoxyliertes Alkanolamin | | | | | 4,5 | |
| Ethoxyliertes Polyethylenimin | | | | | 4,5 | 3,0 |
| Protease | 6 HPE/ml | 6 HPE/ml | 6 HPE/ml | 6 HPE/ml | 6 HPE/ml | 6 HPE/ml |
| Duftstoff(e) | 0,5 | 0,5 | 0,4 | 0,3 | 0,4 | 0,25 |
| DTI, SRP, weitere Enzyme, Entschäumer, u.a. | minors | minors | minors | minors | minors | minors |

**Tabelle 4: Feste Waschmittel**

| **Chemischer Name** | **Gew.-% Aktivsubstanz in der Formulierung** | | |
|---|---|---|---|
| | **A** | **B** | **C** |
| LAS | 12,2 | 12,0 | 10,1 |
| Na-Fettalkoholsulfat, C₁₂₋₁₈ | 4,2 | | |
| Fettalkohol, C₁₂₋₁₈, 7 EO | 4,1 | 2,3 | 1,5 |
| Seifen | 0,4 | | |
| Citrat | 2,0 | | |
| Natriumcarbonat | 2,4 | 17,9 | 25,1 |
| Builder | 23,0 | 7,0 | 7,6 |
| Phosphonat | 1,2 | 1,1 | 1,2 |
| Polyacrylat | 0,12 | 2,8 | 3,0 |
| Carboxymethylcellulose | 2,3 | 2,0 | 1,1 |
| 2Na2 Carbonat 3 H₂O₂ | 18,5 | 15,8 | |
| TAED | 10,9 | 3,5 | |
| Duftstoff(e) | 0,5 | 0,3 | 0,4 |
| Protease | 6 HPE/ml | 6 HPE/ml | 6 HPE/ml |
| Natriumsulfat, Schauminhibitor, optischer Aufheller, Duftstoffe, weitere Enzyme | Rest | Rest | Rest |

**Tabelle 5: Zweiphasiges Geschirrspülmaschinenmittel**

| **Pulverphase (Phase A)** | **A1** | **A2** |
|---|---|---|
| Aktivstoffgehalt in Gew.-% (soweit nichts anderes angegeben), bezogen auf das Gesamtgewicht der Pulverphase | | |
| Natriumpercarbonat | 13,0 | 15,0 |
| Nichtionisches Tenside | 4,0 | 4,0 |
| Sulfonsäuregruppenhaltiges Polymer | 4,0 | 4,0 |
| HEDP (Natriumsalz) | 6,0 | 6,0 |
| Natriumcarbonat (inkl. Natriumhydrogencarbonat) | 24,0 | 28,0 |
| MGDA (Trinatriumsalz) | 0 | 0 |
| Schichtsilikat (SKS 6 Pulver) | 4,0 | 4,0 |
| Natriumcitrat (als wasserfreies Natriumcitrat berechnet) | 21,0 | 21,0 |
| Amylase (Stainzyme^{®} Plus 24 Evity T; Angabe Gew.-% bezogen auf die Menge der eingesetzten Zubereitung, t.q.) | 1,5 | 1,5 |
| Protease (Gesamtaktivprotein) | 40 mg/Job | 40 mg/Job |
| Misc (u.a. Parfüm, Farbstoffe, Konservierungsmittel, Füllstoffe z.B. Natriumsulfat, Bleichkatalysator (MnTACN), Bleichaktivator (TAED), Zinkacetat, Silberschutz, weitere Enzyme) | Add 100 | Add 100 |
| | | |

| **Gelphase (Phase B)** | **B1** | **B2** |
|---|---|---|
| Aktivstoffgehalt in Gew.-% (soweit nichts anderes angegeben), bezogen auf das Gesamtgewicht der Gelphase | | |
| Polymer umfassend Acrylsäure- und Amidopropylsulfonsäurehaltige Monomere | 10,0 | 11,0 |
| Glycerin | 27,0 | 25,0 |
| 1,3-Propandiol | 30,0 | 30,0 |
| PEG 400 | 15,0 | 17,0 |
| PVOH | 15,0 | 14,0 |
| Misc (u.a. Prozesshilfsmittel, pH-Stellmittel, Parfüm, Farbstoff) | Add 100 | Add 100 |
| Gelierzeit / min | weniger als1 | weniger als1 |
| | | |
| Die Phasen A1 bzw. A2 und die Phasen B1 bzw. B2 können beliebig miteinander kombiniert werden. Gesamtgewicht beider Phasen in einer Einmalportion von 18,5 g. | | |

## Patentansprüche

1. Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease, jeweils bezogen auf die Nummerierung gemäß SEQ ID NO:1, an mindestens einer der Positionen, die die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist.

2. Protease nach Anspruch 1, wobei die Protease eine Aminosäuresubstitutionskombination aufweist, die aus der aus (i) A209V-N237P, (ii) A209V, (iii) N121F-A209V-N237P, (iv) A194C-N218IN237W, (v) A209V-N237W und (vi) A209V-N218I-N237Pbestehenden Gruppe ausgewählt ist, jeweils bezogen auf die Nummerierung gemäß SEQ ID NO:1.

3. Protease, **dadurch gekennzeichnet, dass**
(a) sie aus einer Protease nach Anspruch 1 oder 2 als Ausgangsmolekül erhältlich ist durch ein- oder mehrfache konservative Aminosäuresubstitution, wobei die Protease, jeweils bezogen auf die Nummerierung gemäß SEQ ID NO:1, an mindestens einer der Positionen, die die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist;
(b) sie aus einer Protease nach Anspruch 1 oder 2 als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 255, 256, 257, 258, 259, 260, 261, 262, 263, 264, 265, 266, 267, 268 oder 269 zusammenhängenden Aminosäuren mit dem Ausgangsmolekül übereinstimmt, wobei die Protease, jeweils bezogen auf die Nummerierung gemäß SEQ ID NO:1, an mindestens einer der Positionen, die die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist.

4. Verfahren zur Herstellung einer Protease, umfassend das Einbringen mindestens einer Aminosäuresubstitution an mindestens einer der Positionen, die bezogen auf die Nummerierung gemäß SEQ ID NO:1 den Positionen 121, 194, 209, 218 und 237 entsprechen, ausgewählt aus der aus N121F, A194C, A209V, T218I, N237P und N237W bestehenden Gruppe, in ein Ausgangsmolekül, das eine Aminosäuresequenz aufweist, die mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% oder 100% Sequenzidentität mit der in SEQ ID NO:1 angegebenen Aminosäuresequenz über deren Gesamtlänge aufweist.

5. Verfahren nach Anspruch 4, ferner umfassend einen oder mehreren der folgenden Verfahrensschritte:
(a) Einbringen einer ein- oder mehrfachen konservativen Aminosäuresubstitution, wobei die Protease, jeweils bezogen auf die Nummerierung gemäß SEQ ID NO:1, an mindestens einer der Positionen, die den Positionen 121, 194, 209, 218 und 237 entsprechen, mindestens eine Aminosäuresubstitution, die aus der aus N121F, A194C, A209V, T218I, N237P und N237W bestehenden Gruppe ausgewählt ist, aufweist;
(b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 190, 200, 210, 220, 230, 240, 250, 255, 256, 257, 258, 259, 260, 261,262,263, 264, 265, 266, 267, 268 oder 269 zusammenhängenden Aminosäuren mit dem Ausgangsmolekül übereinstimmt, wobei die Protease, jeweils bezogen auf die Nummerierung gemäß SEQ ID NO:1, an mindestens einer der Positionen, die den Positionen 121, 194, 209, 218 und 237 entsprechen, mindestens eine Aminosäuresubstitution, die aus der aus N121F, A194C, A209V, T218lI, N237P und N237W bestehenden Gruppe ausgewählt ist, aufweist.

6. Nukleinsäure codierend für eine Protease nach einem der Ansprüche 1 bis 3 oder codierend für eine nach einem Verfahren der Ansprüche 4 bis 5 erhaltene Protease.

7. Vektor enthaltend eine Nukleinsäure nach Anspruch 6, insbesondere ein Klonierungsvektor oder ein Expressionsvektor.

8. Nicht-menschliche Wirtszelle, die eine Nukleinsäure nach Anspruch 6 oder einen Vektor nach Anspruch 7 beinhaltet, oder die eine Protease nach einem der Ansprüche 1 bis 3 beinhaltet, oder die eine nach einem Verfahren der Ansprüche 4 bis 5 erhaltene Protease beinhaltet, insbesondere eine, die die Protease in das die Wirtszelle umgebende Medium sezerniert.

9. Verfahren zur Herstellung einer Protease umfassend
a) Kultivieren einer Wirtszelle gemäß Anspruch 10 und
b) Isolieren der Protease aus dem Kulturmedium oder aus der Wirtszelle.

10. Mittel, insbesondere Wasch- oder Reinigungsmittel, insbesondere Textilwaschmittel, **dadurch gekennzeichnet, dass** es mindestens eine Protease nach einem der Ansprüche 1 bis 3 oder eine nach einem Verfahren der Ansprüche 4 bis 5 erhaltene Protease enthält.

11. Verfahren zur Reinigung von Textilien und/oder harten Oberflächen, insbesondere Geschirr, **dadurch gekennzeichnet, dass** in mindestens einem Verfahrensschritt ein Mittel nach Anspruch 10 angewendet wird.

12. Verwendung einer Protease nach einem der Ansprüche 1 bis 3 oder eine nach einem Verfahren der Ansprüche 4 bis 5 erhaltene Protease in einem Wasch- oder Reinigungsmittel, insbesondere Textilwaschmittel zur Entfernung mindestens einer proteasesensitiven Anschmutzung.
